# EUROPEAN PATENT APPLICATION

(11) **EP 4 596 107 A1**
(43) Date of publication of application: **06.08.2025**
(21) Application number: 23872725.9
(22) Date of filing: 03.07.2023
(51) Int. Cl.: B01L 7/00

(54) **GENOME AMPLIFICATION MODULE HAVING BRANCH SPACE ADJACENT TO EXTRACT INLET**

(30) Priority: 28.09.2022 KR 20220123435
(71) Applicant: SD Biosensor, Inc., Gyeonggi-do 16690 (KR)
(72) Inventor: CHO, Young-Shik, Suwon-si, Gyeonggi-do 16690 (KR); LEE, Sunyoung, Suwon-si, Gyeonggi-do 16693 (KR); LIM, Kwanhun, Daejeon 35215 (KR); KIM, Dong-Hun, Suwon-si, Gyeonggi-do 16700 (KR); KIM, In-Ae, Suwon-si, Gyeonggi-do 16697 (KR); PARK, Hyo-Lim, Suwon-si, Gyeonggi-do 16685 (KR); LA, Sun-Hyuk, Uijeongbu-si, Gyeonggi-do 11712 (KR)
(74) Representative: J A Kemp LLP
(86) International application number: PCT/KR2023/009297
(87) International publication number: WO 2024/071599

(57) **Abstract**

The present invention relates to a genome amplification module having a branch space adjacent to an inlet through which an extract is introduced, the genome amplification module in which a branch space is formed in a portion adjacent to an inlet and is wider and deeper than an extract moving passage so that the extract can be simultaneously injected along each extract moving passage after the extract is filled in the branch space.

## Description

### [Technical Field]

The present invention relates to a genome amplification module having a branch space adjacent to an inlet through which an extract is introduced.

### [Background Art]

In modern times, advancements in biotechnology have made it possible to interpret the causes of diseases at the genetic level. Thus, there is an increasing demand for manipulation and biochemical analysis of biological samples for curing or preventing human diseases.

Additionally, technology for extracting and analyzing nucleic acids from biological samples or samples containing cells is required in various fields such as new drug development, pre-test for virus or bacterial infection, and forensics in addition to disease diagnosis.

Meanwhile, Korean Patent Registration No. 10-2362853 discloses an extraction device for preparing an extract containing a genome by pre-treating an injected sample, as a device developed by the present applicant. Extract generated by the extraction device moves to an amplification module connected to the extraction device, and the extract injected into a receptacle of the amplification module is amplified through a nucleic acid amplification reaction. A probe that specifically binds to a target sequence and contains a fluorescent substance is stored in the receptacle, so that, when the target sequence is included in the genome of the extract, fluorescence can be observed through the nucleic acid amplification reaction. In addition, depending on whether fluorescence is observed, whether the individual from which the sample was collected is infected with a disease/virus can be determined.

Meanwhile, in the case of an amplification module having a plurality of receptacles, primers/probes for detecting different targets may be stored in each receptacle. When the extracts injected into the plurality of receptacles are mixed during the injection/amplification process, inaccurate detection results will be obtained. In addition, to ensure relatively uniform results, the same amount of extract needs to be injected into each receptacle to; however, in conventional genome amplification modules, a greater volume of extract tend to be injected into the receptacle that is relatively less affected by gravity.

Thus, the inventors of the present invention devised and completed the present invention to address and resolve the problems of such conventional genome amplification modules according to the related art.
(Patent document 1) Korean Patent Registration No. 10-2346703 (December 29, 2021)
(Patent document 2) Korean Patent Registration No. 10-2416335 (June 29, 2022)
(Patent document 3) Korean Patent Registration No. 10-2293717 (August 19, 2021)
(Patent document 4) Korean Patent Registration No. 10-2375252 (March 11, 2022)
(Patent document 5) Korean Patent Registration No. 10-2362853 (February 9, 2022)

### [Disclosure]

### [Technical Problem]

According to the present invention, the present invention provides a genome extraction device in which an inner chamber containing reagents required for genome extraction is provided separately from an outer chamber and as upper and lower parts of the inner chamber are sealed, a problem that the reagents accommodated in a single chamber leak out in a genome extraction device according to the related art can be solved.

The present invention also provides a genome extraction device including a safety clip for preventing a sealing member sealing upper and lower openings of an inner chamber from being perforated by protrusion members formed in a cover and the outer chamber by the inner chamber moving up and down due to vibration that occurs during a production and distribution process of a product.

The present invention also provides a genome extraction device that solves the problem of cross-contamination between reagents by a capillary phenomenon occurring through a space between double chambers through a unique inner chamber design (lower inner chamber).

The present invention also provides a genome extraction device that solves the problem of reagents leaking out through a unique inner chamber design (upper inner chamber) in a structure for preventing a capillary phenomenon.

The present invention also provides a genome extraction device in which, due to the configuration of a first protrusion member formed on a bottom surface of an outer chamber, a sealing member can be torn with a small force and a perforated portion is expanded so that a reagent accommodated inside the inner chamber can smoothly flow out to the outside.

The present invention also provides a genome extraction device in which an inclined portion is formed around a discharge hole through which reagents are discharged, so that the reagents can smoothly flow out through the discharge hole.

The present invention also provides a genome extraction device in which a double-structured flow cover-pad is arranged between an outer chamber and a base plate so that the convenience of manufacturing is improved and the problem of a flow path being unintentionally narrowed is solved, compared to a genome extraction device according to the related art in which only one pad is arranged.

The present invention also provides a genome extraction device in which firm coupling between a base plate-flow cover-pad-outer chamber is achieved so that sealed flow paths are formed without a phenomenon of reagents flowing out in the middle during the movement process of the reagents.

The present invention also provides a genome extraction device in which a bead chamber in which beads required for genome extraction and amplification are accommodated, also has a double chamber structure of an outer chamber and a bead chamber so that the performance of beads vulnerable to moisture can be maintained for a long time.

The present invention also provides a genome extraction device in which, even if a bead chamber is opened, the performance of beads is maintained by having a dehumidifying portion located at the upper part of the bead chamber.

The present invention also provides a genome extraction device in which, as a pre-treated extract is injected, air remaining inside a receptacle is easily discharged so that an amplification module into which a sufficient capacity of the extract can be injected, is applied to the genome extraction device.

The present invention also provides a genome extraction device in which an amplification module has a plurality of receptacles and primers and probes for different genome amplifications are stored in each receptacle so that various types of diseases can be diagnosed through one genome extraction.

The present invention also provides a genome amplification module in which a plurality of extract moving passages have the same capacity (volume) so that extracts can be simultaneously injected into all receptacles and the same amount of extract can be injected.

The present invention also provides a genome amplification module in which a branch space is formed in a portion adjacent to an inlet and is wider and deeper than an extract moving passage so that extracts can be simultaneously injected along each extract moving passage after the branch space is filled with the extracts.

The present invention also provides a genome amplification module in which a branch space is formed in a first end adjacent to an inlet and a receptacle is provided in a second end far from the first end so that an amplification product in each receptacle can be prevented from being pushed out to a moving passage by a heating temperature or flowing back to another receptacle during an amplification process.

The present invention also provides a genome amplification module in which a space is formed in a connection point of a receptacle of a gas moving passage and is wider and deeper than other portions so that, even if bubbles are generated, they do not affect the receptacle and thus detection accuracy can be improved.

The present invention also provides a genome amplification module in which the width and depth of a gas moving passage are provided to a minimum so that an extract discharged through the gas moving passage is minimized.

The present invention also provides a genome extraction method using the aforementioned genome extraction device.

### [Technical Solution]

According to an aspect of the present invention, there is provided an amplification module including a body, an inlet which is formed in the body and through which an extract is introduced, a plurality of receptacles which are connected to the inlet and in which the introduced extract is accommodated, a first branch space which communicates with the inlet, a plurality of extract moving passages which are branched from the first branch space and connect the inlet and the plurality of receptacles to each other, an outlet which is formed in the body and through which gas is discharged, and a plurality of gas moving passages which connect the outlet and the plurality of receptacles to each other.

The first branch space may include a first penetration portion which penetrates the body while being connected to the inlet, and a first recessed portion which is formed between the first penetration portion and the plurality of extract moving passages and recessed in one surface of the body.

One or more of the width and depth of the first branch space may be wider or deeper than the width and depth of the extract moving passages.

Volumes of the plurality of extract moving passages may be all the same.

The first branch space may be formed in a first end of the amplification module in a width direction, and the plurality of receptacles may be formed in a second end opposite to the first end of the amplification module in the width direction.

The amplification module may further include a second branch space which is formed between the outlet and the plurality of gas moving passages, wherein the second branch space includes a second penetration portion which penetrates the body while being connected to the outlet, and a second recessed portion which is formed between the second penetration portion and the plurality of gas moving passages and recessed in the other surface of the body.

The first recessed portion and the second recessed portion may have a portion where they overlap partially in the width direction of the body and are recessed in the body.

The amplification module may further include a sealing member which is attached to one surface of the body and an opposite surface to the one surface and is configured to seal the plurality of receptacles, the first branch space, the second branch space, the extract moving passages and the gas moving passages from an external space.

A space between the first recessed portion and the sealing member and a space between the second recessed portion and the second sealing member may not communicate with each other.

The gas moving passage may be connected to an upper part of the receptacle, and a space may be formed in a connection point with the receptacle of the gas moving passage and may be wider and deeper than other portions.

One or more of the width and depth of the gas moving passage may be narrower or shallower than the width and depth of the extract moving passage.

The gas moving passage may be formed through a combination of one or more including a first gas moving passage having a first width and a first depth, a second gas moving passage having a second width greater than the first width and the first depth, and a third gas moving passage having a third width greater than the second width and a second depth deeper than the first depth.

A plurality of pillars may protrude from the gas moving passage.

Gas moving passages connected to the plurality of receptacles may have the same capacity.

Probes and primers for amplifying a first target material may be stored in one of the plurality of receptacles, and probes and primers for amplifying a second target material different from the first target material may be stored in another receptacle.

### [Advantageous Effects]

In a genome extraction device according to the present invention, an inner chamber containing reagents required for genome extraction is provided separately from an outer chamber and as upper and lower parts of the inner chamber are sealed, a problem that the reagents accommodated in a single chamber leak out in a genome extraction device according to the related art can be solved.

In addition, the inner chamber moves up and down due to vibration that occurs during a production and distribution process of a product, so that a sealing member sealing upper and lower openings of an inner chamber can be prevented from being perforated by protrusion members formed in a cover and the outer chamber.

In addition, the problem of cross-contamination between reagents can be solved by a capillary phenomenon occurring through a space between double chambers.

In addition, reagents can be prevented from leaking out to the outside in a structure for preventing a capillary phenomenon.

In addition, due to the configuration of a first protrusion member formed on a bottom surface of an outer chamber, a sealing member can be torn with a small force and a perforated portion is expanded so that a reagent accommodated inside the inner chamber can smoothly flow out to the outside.

In addition, an inclined portion is formed around a discharge hole through which the reagents are discharged, so that the reagents can smoothly flow out through the discharge hole.

In addition, a double-structured flow cover-pad is arranged between an outer chamber and a base plate so that in which the convenience of manufacturing is improved and the problem of a flow path being unintentionally narrowed is solved, compared to a genome extraction device according to the related art in which only one pad is arranged.

In addition, firm coupling between a base plate-flow cover-pad-outer chamber is achieved so that sealed flow paths are formed without a phenomenon of reagents flowing out in the middle during the movement process of the reagents.

In addition, a bead chamber in which beads required for genome extraction and amplification are accommodated, also has a double chamber structure of an outer chamber and a bead chamber so that the performance of beads vulnerable to moisture can be maintained for a long time.

Even if the bead chamber is opened, the performance of beads is maintained by having a dehumidifying portion located at the upper part of the bead chamber.

As a pre-treated extract is injected, air remaining inside a receptacle is easily discharged so that a sufficient capacity of the extract can be injected into the amplification module.

In addition, an amplification module has a plurality of receptacles and primers and probes for different genome amplifications are stored in each receptacle so that various types of diseases can be diagnosed through one genome extraction.

In addition, a plurality of extract moving passages have the same capacity (volume) so that extracts can be simultaneously injected into all receptacles and the same amount of extract can be injected.

In addition, a branch space is formed in a portion adjacent to an inlet and is wider and deeper than an extract moving passage so that extracts can be simultaneously injected along each extract moving passage after the branch space is filled with the extracts.

In addition, a branch space is formed in a first end adjacent to an inlet and a receptacle is provided in a second end far from the first end so that an amplification product in each receptacle can be prevented from being pushed out to a moving passage by a heating temperature or flowing back to another receptacle during an amplification process.

In addition, a space is formed in a connection point of a receptacle of a gas moving passage and is wider and deeper than other portions so that, even if bubbles are generated, they do not affect the receptacle and thus detection accuracy can be improved.

In addition, the width and depth of a gas moving passage are provided to a minimum so that an extract discharged through the gas moving passage is minimized.

### [Brief Description of the Drawing]

FIG. 1 is a perspective view of the overall appearance of a genome extraction device according to an embodiment of the present invention;
FIG. 2 is a perspective view of the genome extraction device of FIG. 1 from another angle;
FIG. 3 is an exploded perspective view of FIG. 1;
FIG. 4 is a diagram illustrating the coupling relationship between an outer chamber and an inner chamber;
FIG. 5 is a diagram illustrating the coupling relationship between an inner chamber and a safety clip;
FIG. 6 is a plan view of the outer chamber;
FIG. 7 is a cross-sectional view illustrating the coupling relationship between the inner chamber and the outer chamber;

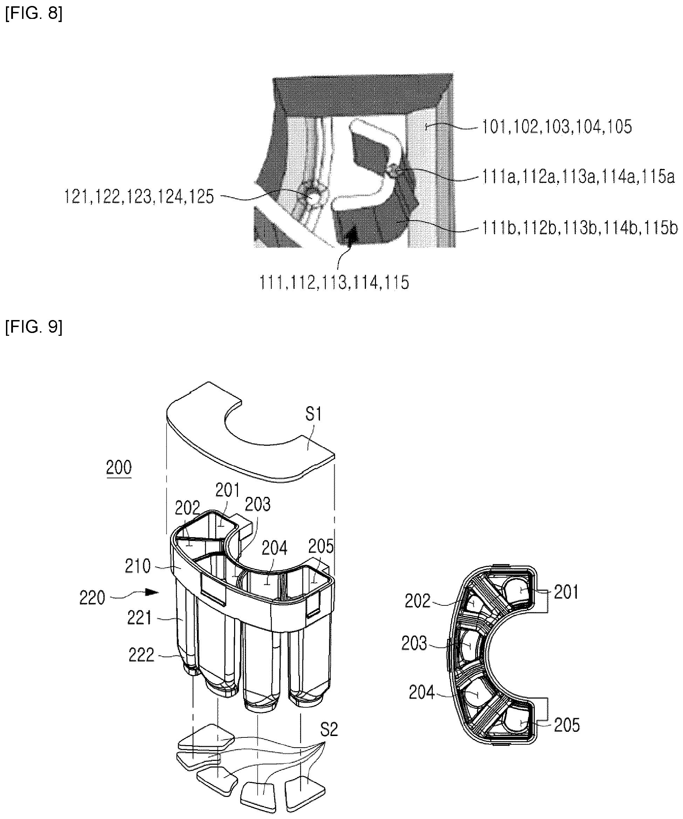

FIG. 8 is an enlarged view illustrating a second protrusion member formed on a bottom surface of the outer chamber;
FIG. 9 is a diagram illustrating the inner chamber in more detail;
FIG. 10 is a bottom perspective view illustrating a cover in more detail;
FIG. 11 is an exploded perspective view illustrating a flow cover and a pad disposed between a base plate and the outer chamber in more detail;
FIG. 12 is an exploded perspective view illustrating configurations of a piston in detail;
FIG. 13 is a bottom perspective view of a flow cover;
FIG. 14 is a perspective view illustrating a base plate in more detail;
FIG. 15 is a cross-sectional view illustrating a genome extraction device according to an embodiment of the present invention in detail;

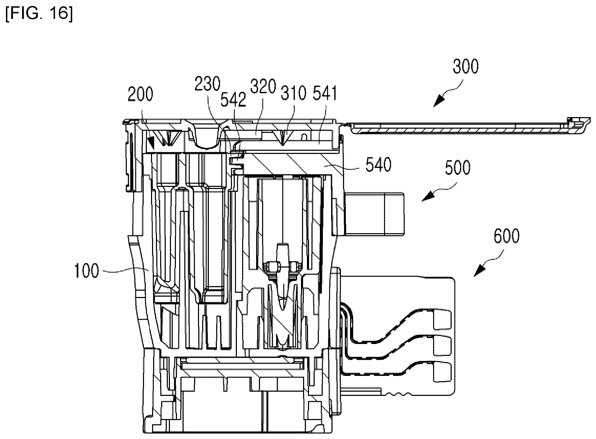

FIG. 16 is another cross-sectional view illustrating a genome extraction device according to an embodiment of the present invention in detail;
FIGS. 17 to 20 are diagrams illustrating an amplification module according to a first embodiment of the present invention;
FIGS. 21 to 23 are diagrams illustrating an amplification module according to a second embodiment of the present invention;
FIGS. 24 to 26 are diagrams illustrating an amplification module according to a third embodiment of the present invention;
FIGS. 27 to 29 are diagrams illustrating an amplification module according to a fourth embodiment of the present invention;
FIG. 30 is a plan view of a bead chamber;
FIGS. 31 and 32 are perspective views illustrating the configuration of a bead chamber in more detail;
FIG. 33 is a latitudinal cross-sectional view of the bead chamber of FIG. 31; and
FIG. 34 is a longitudinal cross-sectional view of the bead chamber of FIG. 31 and is a diagram illustrating a structure when it is with the outer chamber.

### [Detailed Description of Preferred Embodiments]

In some cases, in order to avoid ambiguity in the concept of the present invention, known structures and apparatus may be omitted or illustrated in block diagram form focusing on the core functions of each structure and apparatus.

Throughout the specification, when a part is said to "comprising" a certain component, this does not mean excluding other components unless otherwise specifically stated, but rather includes other components. In addition, terms such as "... part", "... unit", and "module" described in the specification mean a unit that processes at least one function or operation, which may be implemented by hardware or software or a combination of hardware and software. In addition, "a or an", "one", "the" and similar related words may be used in the context of describing the invention (especially in the context of the claims below) to include both singular and plural meanings, unless otherwise indicated in the present specification or clearly contradicted by the context.

In describing embodiments of the present invention, if it is determined that a specific description of a known function or configuration may unnecessarily obscure the gist of the present invention, the detailed description thereof will be omitted. And the terms described below are terms defined in consideration of functions in the embodiments of the present invention, and may vary depending on the intention or custom of the user or operator. Thus, the definitions should be made based on the contents throughout this specification.

Hereinafter, the present invention will be described in detail with reference to the accompanying drawings.

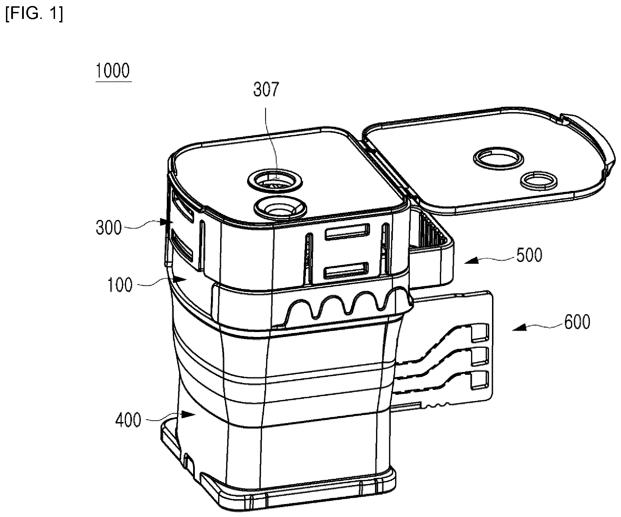

Referring to FIGS. 1 through 3, a genome extraction device 1000 according to an embodiment of the present invention includes an outer chamber 100, an inner chamber 200, a cover 300, a base plate 400, a safety clip 500, an amplification module 600, a piston 700, a driving unit 800, and a bead chamber 900.

The outer chamber 100 is partitioned into a plurality of first spaces 101, 102, 103, 104, 105, 106, and 107 by an outer chamber partition wall. That is, the plurality of first spaces 101, 102, 103, 104, 105, 106, and 107 may be mutually independent spaces.

The plurality of first spaces 101, 102, 103, 104, 105, 106, and 107 may have an open upper portion and a closed lower portion. On the other hand, first discharge holes 121, 122, 123, 124, and 125 are formed through the bottom surface of the plurality of first spaces 101, 102, 103, 104, and 105 along a circumferential direction while being distanced apart by a first distance from the central portion of the outer chamber 100, and second discharge holes 126 and 127 are formed through the bottom surface of the remaining first spaces 106 and 107 along the circumferential direction while being distanced apart by a second distance from the central portion of the outer chamber 100. In addition, discharge holes 128 and 129 are formed through the bottom surface of a space between the first spaces 106 and 107 and communicate with the amplification module 600. Here, the first distance may be shorter than the second distance, but in another embodiment, the first distance may be longer than the second distance.

Reagents stored in the inner chamber 200 to be described later are placed into the plurality of first spaces 101, 102, 103, 104, and 105, and beads stored in the bead chamber 900 are placed into the remaining first spaces 106 and 107.

A piston insertion portion 108 into which the piston 700 is inserted is vertically formed through the center of the plurality of first spaces 101, 102, 103, 104, 105, 106, and 107. The piston 700 is inserted into the piston insertion portion 108, and a driving unit (not shown) of a diagnostic device is coupled to the piston 700 to elevate the piston 700 so that reagents (fluids) in the first spaces 101, 102, 103, 104, 105, 106, and 107 may enter and exit a fluid receptacle 701 inside the piston 700. More specific details will be described later.

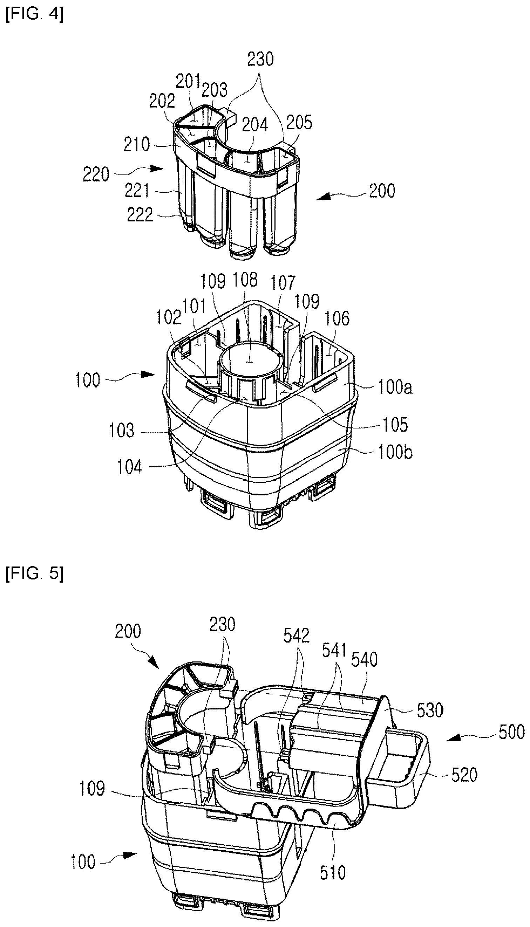

Referring to FIG. 4, an outer surface upper portion 100a of the outer chamber 100 is recessed toward the center while being connected to an upper part of an outer surface lower portion 100b. The safety clip 500 may be coupled to the outer surface upper portion 100a of the outer chamber 100, and a boundary between the outer surface upper portion 100a and the outer surface lower portion 100b serves as a step for the safety clip 500, ensuring that once the safety clip 500 is coupled to the outer surface upper portion 100a, its coupling position remains secured. The safety clip 500 includes an outer chamber coupling portion 510 that extends while having a length at least partially surrounding the outer surface upper portion 100a of the outer chamber 100, and a handle 520 formed at one side of the outer chamber coupling portion 510.

As the safety clip 500 is coupled to the outer chamber 100, the cover 300 presses the inner chamber 200 coupled to the outer chamber 100 so that upper and lower openings of the inner chamber 200 may be prevented from being opened. It is possible for a user to start an extraction process after removing the safety clip 500 from the outer chamber 100 by gripping the handle 520. In other words, when the safety clip 500 is coupled to the outer chamber 100, the reagents in the inner chamber 200 cannot be introduced into the outer chamber 100. Only when the safety clip 500 is removed from the outer chamber 100, the reagents in the inner chamber 200 can be introduced into the outer chamber 100.

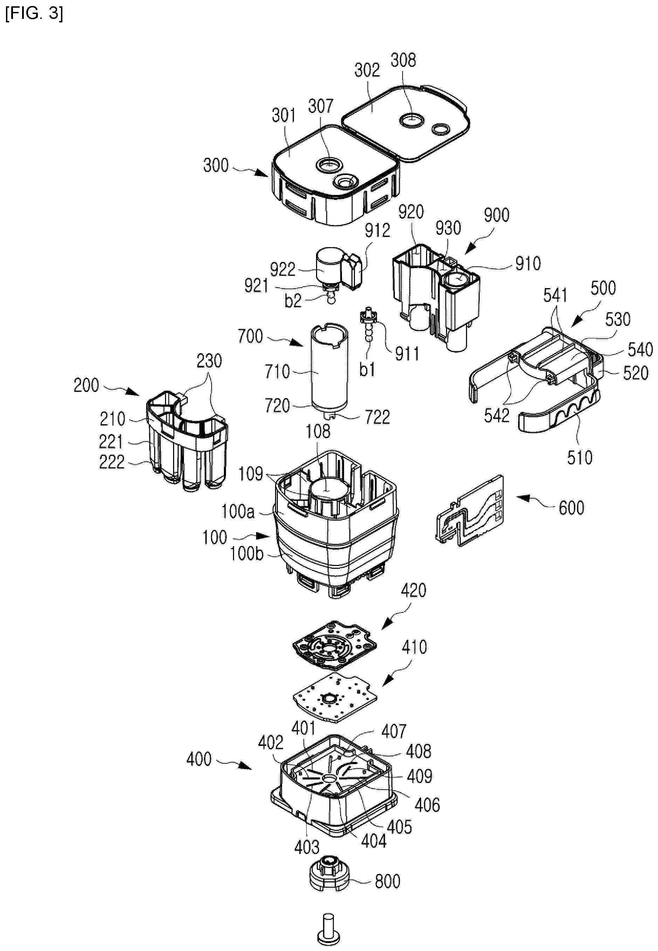

Referring to FIGS. 3 through 5, the configuration of the safety clip 500 will be described in more detail.

The safety clip 500 includes an outer chamber coupling portion 510, the handle 520, an upper extension portion 530, and a side extension portion 540.

The outer chamber coupling portion 510 is coupled to the outer chamber 100 while surrounding at least partially an outer circumference (specifically, the outer surface upper portion 100a) of the outer chamber 100. More specifically, the outer chamber coupling portion 510 is coupled to the outer chamber 100 to surround four outer surfaces of the outer chamber 100, and extension ends of the outer chamber coupling portion 510 may be configured to be spaced apart from each other. As shown in FIG. 1, when the safety clip 500 is coupled to the outer chamber 100, the extension ends of the outer chamber coupling portion 510 are caught on one of the outer surfaces of the outer chamber 100 so that the user needs to grip the safety clip 500 and apply an external force in one direction to separate the safety clip 500 from the outer chamber 100.

The handle 520 is a portion that extends outwards from the outer chamber coupling portion 510 and is a portion that is gripped by the user to separate the safety clip 500 from the outer chamber 100.

The upper extension portion 530 extends upwards from one side of the outer chamber coupling portion 510, and the side extension portion 540 extends from the upper extension portion 530 toward the center of the outer chamber 100.

The safety clip 500 according to the embodiment of the present invention is characterized in that a cover support member 541 protrudes from the upper surface of the side extension portion 540 and an inner chamber coupling portion 542 protrudes from the extended end of the side extension portion 540. Thereby, the cover support member 541 prevents the protrusion members 311, 312, 313, 314, 315, 316, and 317 formed on the bottom surface of the cover 300 from tearing (perforating) a first sealing member S1, which seals upper openings of a plurality of second spaces 201, 202, 203, 204, and 205 of the inner chamber 200 and a third sealing member S3, which seals an upper opening of the bead chamber 900 when the safety clip 500 is coupled to the outer chamber 100.

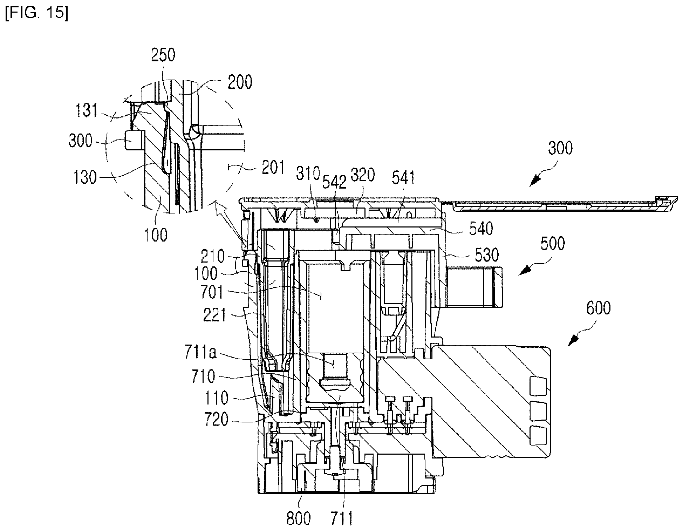

As the cover support member 541 protrudes upwards from the upper surface of the side extension member 540, when the safety clip 500 is coupled to the outer chamber 100 and the inner chamber 200, as shown in FIG. 15, contact between the protrusion members 311, 312, 313, 314, 315, 316, and 317 and the first sealing member S1 and the third sealing member S3 is blocked. Thus, when the safety clip 500 is coupled to the outer chamber 100 and the inner chamber 200, it prevents perforation of the inner chamber 200 and the bead chamber 900. As a result, the leakage of the reagent accommodated in the inner chamber 200 and beads accommodated in the bead chamber 900 into the outer chamber 100 is prevented.

The inner chamber coupling portion 542 is a portion that is coupled to the fixing portion 230 of the inner chamber 200 when the safety clip 500 is coupled to the outer chamber 100. When the inner chamber coupling portion 542 is coupled to the fixing portion 230, the bottom surface of the inner chamber 200 is positioned at a position spaced apart from the bottom surface of the outer chamber 100 by a predetermined distance. Therefore, the second sealing member S2, which seals the lower openings of the plurality of second spaces 201, 202, 203, 204, and 205 is prevented from being torn by the protrusion members 111, 112, 113, 114, and 115 formed on the bottom surface of the outer chamber 100 (see FIG. 15).

In the attached drawing, the inner chamber coupling portion 542 is illustrated in the form of a coupling projection, and the fixing portion 230 is illustrated in the form of a coupling groove that engages with the coupling projection, but in other embodiments, the inner chamber coupling portion 542 may be provided in the form of a coupling groove, and the fixing portion 230 may be provided in the form of a coupling projection that engages with the coupling groove.

A mounting portion 109, that provides a space in which the fixing portion 230 of the inner chamber 200 is mounted, may be formed by being recessed in the outer chamber 100 (more specifically, the outer chamber partition wall). While the inner chamber 200 is fixed at a position spaced apart from the bottom surface of the outer chamber 100 by a coupling structure with the safety clip 500, a fixing force may be further enhanced as the fixing portion 230 of the inner chamber 200 is mounted on and supported by the mounting portion 109.

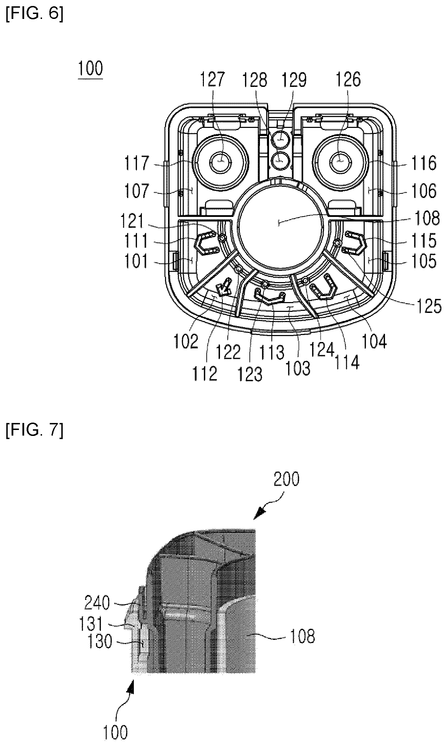

Referring to FIG. 7, an insertion space 130 is recessed in the upper side of the inner wall of the outer chamber 100, and a coupling hook 240 of the inner chamber 200 may be coupled to the insertion space 130. A stopper 131 protrudes from the upper side of the insertion space 130 toward the inside of the outer chamber 100. Thus, when the inner chamber 200 is not pressed by the cover 300, the coupling hook 240 of the inner chamber 200 is positioned on the stopper 131, but when the inner chamber 200 is pressed by the cover 300, the coupling hook 240 may pass the stopper 131 and be inserted into the insertion space 130.

Referring to FIG. 15, an outer chamber-inner chamber coupling relationship according to another embodiment of the present invention will be described. In FIG. 7, instead of the coupling hook 240 formed in the inner chamber 200, a catch projection 250 protruding outwards from the outer wall of the inner chamber 200 is provided, and the catch projection 250 is caught by a stopper 131 formed in the inner wall of the outer chamber 100, thereby partially restricting downward movement of the inner chamber 200. When the safety clip 500 is removed from the outer chamber 100 and the inner chamber 200 is pressed by the cover 300, the catch projection 250 passes the stopper 131 and is inserted into the insertion space 130, and thus, the second sealing member S2 which seals the plurality of second spaces of the inner chamber 200 is perforated by the protrusion members formed in the outer chamber 100.

The inner chamber 200 is partitioned into a plurality of second spaces 201, 202, 203, 204, and 205 by an inner chamber partition wall. That is, the plurality of second spaces 201, 202, 203, 204, and 205 may be independent spaces.

The upper and lower portions of the plurality of second spaces 201, 202, 203, 204, and 205 are open (that is, the plurality of second spaces have an upper opening and a lower opening), and the upper portion is sealed by a first sealing member S1, and the lower portion is sealed by a second sealing member S2. The first sealing member S1 and the second sealing member S2 may be, for example, a film, but the present invention is not limited thereto. The first sealing member may be made of any material that does not allow fluid to pass through.

Different reagents may be injected into each of the plurality of second spaces 201, 202, 203, 204, and 205. The second sealing member S2 seals the lower portion of the plurality of second spaces, before the reagents are injected. After the reagents are introduced, the first sealing member S1 seals the upper portion of the plurality of second spaces, thereby completing the injection of the reagents into the inner chamber 200.

Referring to FIG. 4, the inner chamber 200 includes an upper inner chamber 210 and a lower inner chamber 220.

The upper inner chamber 210 is formed integrally and is configured to be in close contact with the inner wall of the outer chamber 100 when the upper inner chamber 210 is coupled with the outer chamber 100.

The lower inner chamber 220 is connected to the upper inner chamber 210, and includes a curved portion (toward the radial inward direction) so as to be separated from the inner wall of the outer chamber 100 when the lower inner chamber 220 is coupled with the outer chamber 100.

Since a dual chamber structure including an inner chamber and an outer chamber is used in the present invention, there may be a risk of cross-contamination with reagents in the inner chamber 200 during operation. Cross-contamination may occur due to a capillary phenomenon through the microscopic space between the inner chamber and the outer chamber. In order to prevent the cross-contamination problem, the present invention has a configuration in which the inner chamber 200 is sufficiently separated and spaced apart from the inner wall of the outer chamber 100 by way of, for example, adopting a curved structure, thereby preventing the capillary phenomenon.

In addition, in order to prevent reagent leakage through the spaced section between the outer chamber 100 and the inner chamber 200 apart to prevent the capillary phenomenon, the upper inner chamber 210 is configured to be in close contact with the inner wall of the outer chamber 100.

On the other hand, first protrusion members 111, 112, 113, 114, and 115 protrude from the bottom surface of the plurality of first spaces 101, 102, 103, 104, and 105 by tearing the second sealing member S2 of the inner chamber 200, thereby allowing the reagent accommodated in the inner chamber 200 to leak out into the plurality of first spaces 101, 102, 103, 104, and 105.

Each of the first protrusion members 111, 112, 113, 114, and 115 may be arranged to correspond one-to-one with the plurality of first spaces 101, 102, 103, 104, and 105. For example, the first protrusion member corresponding to reference numeral 111 tears the second sealing member S2 which seals the upper part of the first space corresponding to reference numeral 101, and the first protrusion member corresponding to reference numeral 115 tears the second sealing member S2 which seals the upper part of the first space corresponding to reference numeral 105.

The first protrusion members 111, 112, 113, 114, and 115 include protrusions 111a, 112a, 113a, 114a, and 115a that protrude from the bottom surface of the plurality of first spaces 101, 102, 103, 104, and 105 by a first height h1, and wing portions 111b, 112b, 113b, 114b, and 115b that extend from the protrusions 111a, 112a, 113a, 114a, and 115a and protrude from the bottom surface by a second height h2 that is lower than the first height h1. Here, the wing portions 111b, 112b, 113b, 114b, and 115b may be structures extending in both left and right directions from the protrusions 111a, 112a, 113a, 114a, and 115a.

The protrusions perform the function of perforating the second sealing member S2, and the wing portions perform the function of expanding the perforation portion of the second sealing member S2. In the present invention, since the height of the protrusions is higher than that of the wing portions, point-contact is made between the second sealing member S2 for sealing the lower part of the inner chamber 200 and the protrusions, and the pressure is minimized when the second sealing member S2 is torn through the point-contact. Thus, the second sealing member S2 may be torn with less force.

When the second sealing member S2 is torn by the protrusion members 111, 112, 113, 114, and 115, the reagents stored in the plurality of second spaces 201, 202, 203, 204, and 205 of the inner chamber 200 flow out into the plurality of first spaces 101, 102, 103, 104, and 105 of the outer chamber 100. Then, the flowed out reagents are discharged through the first discharge holes 121, 122, 123, and 124 formed on the bottom surface of the first spaces 101, 102, 103, 104, and 105. In order to facilitate discharge of the reagents into the first discharge holes 121, 122, 123, 124, and 125, the areas around the first discharge holes 121, 122, 123, 124, and 125 are provided with portions inclined downward to the first discharge holes 121, 122, 123, 124, and 125. The inclined portions may have an angle of 3 to 10 degrees, and thus, the reagents that have flowed into the first spaces 101, 102, 103, 104, and 105 can be more easily discharged through the first discharge holes 121, 122, 123, 124, and 125.

The cover 300 is configured to be coupled to the upper portion of the outer chamber 100 and to cover the upper portions of the outer chamber 100 and the inner chamber 200.

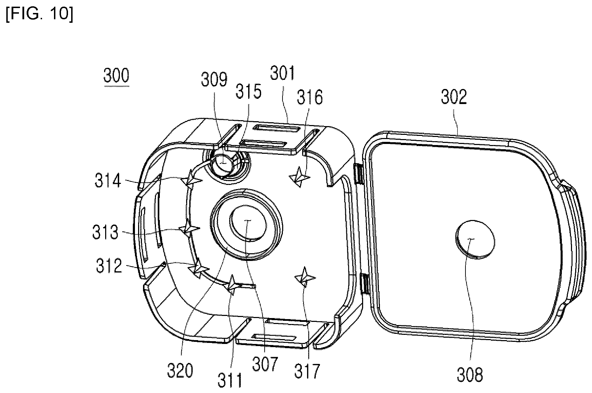

Referring to FIG. 10, the cover 300 includes a cover body 301 and a lid 302.

A first insertion hole 307 aligned with the piston insertion portion 108 and a first specimen injection hole 309 into which a specimen is injected are formed through the cover body 301, and second protrusion members 311, 312, 313, 314, and 315 for tearing the first sealing member S1 and third protrusion members 316 and 317 for tearing the third sealing member S3 protrude from the bottom surface of the cover body 301.

The second protrusion members 311, 312, 313, 314, and 315 may be arranged to correspond one-to-one with the plurality of first spaces 101, 102, 103, 104, 105, 106, and 107, and the third protrusion members 316 and 317 may be arranged to correspond one-to-one with a plurality of third spaces 910 and 920. For example, the second protrusion member corresponding to reference numeral 311 tears the first sealing member S1 for sealing the upper opening of the first space corresponding to reference numeral 101, and the second protrusion member corresponding to reference numeral 315 tears the first sealing member S1 for sealing the upper opening of the first space corresponding to reference numeral 105.

A separation member 320 is formed on the bottom surface of the cover body 301 along the periphery of the first insertion hole 307. The separation member 320 is a portion that allows the first protrusion member and the first sealing member to be separated from each other when the safety clip 500 is coupled to the outer chamber 100. That is, as the separation member 320 is supported by the cover support member 541, the cover 300 is separated from the inner chamber 100 by a predetermined distance.

The lid 302 is connected to one side of the cover body 301 so as to be hingerotatable. A second insertion hole 308 that is aligned with the first insertion hole 307 is formed through the central portion of the lid 302.

When the safety clip 500 is separated from the outer chamber 100 in a state in which the cover 300 is coupled to the outer chamber 100 and then the cover 300 is pressed downwards, the inner chamber 200 coupled to the outer chamber 100 descends along the inner wall of the outer chamber 100. Since first protrusion members 111, 112, 113, 114, 115, 116, and 117 are formed on the bottom surface of the outer chamber 100, and second protrusion members 311, 312, 313, 314, and 315 and third protrusion members 316 and 317 are formed on the bottom surface of the cover 300, the first sealing member S1 and the second sealing member S2 that seal the upper and lower openings of the inner chamber 200 and the third sealing member S3 that seals the upper opening of the bead chamber 900 are torn by the protrusion members. Thus, the reagents accommodated in the inner chamber 200 flow out into the plurality of first spaces 101, 102, 103, 104, and 105 of the outer chamber 100. As the second sealing member S2 for sealing the upper opening of the inner chamber 200 is torn, it functions as an air vent so that the reagents may be sufficiently discharged into the first space.

The base plate 400 is coupled to the lower portion of the outer chamber 100 and includes a plurality of paths that guide a path along which the reagents move between the first spaces 101, 102, 103, 104, 105, 106, and 107 of the outer chamber 100 and the fluid receptacle of the piston 700.

According to an embodiment of the present invention, the base plate 400 may have a liquid flow path through which liquid may move and an air flow path through which air may move. The base plate 400 may further include a flow cover 410 and a pad 420 that is between the outer chamber 100 and the base plate 400 and disposed on the upper surface of the base plate 400 to prevent leakage of liquid when coupled with the outer chamber 100. When the base plate 400-the flow cover 410-the pad 420 are assembled, the liquid flow path and the air flow path of the base plate 400 are sealed on their upside by the flow cover 410 and the pad 420, forming a space and completing a perfect path.

The liquid flow path is connected to the flow cover 410, the pad 420, and the outer chamber 100 to provide a space through which a specimen and a reagent may move and be mixed.

The air flow path connects a vacuum control section of the amplification module 600 and the piston 700, thereby controlling the vacuum that may occur when the extracted genome moves to the amplification module 600. Additionally, it prevents contamination of an amplification product that may arise during the amplification of the genome.

A plurality of flow paths 401, 402, 403, 404, 405, 406, 407, 408, and 409 are formed on the upper portion of the base plate 400. Each flow path does not intersect with each other, and is formed to extend from the center to the outer part of the base plate 400. Here, the liquid flow paths correspond to reference numerals 401 to 408, while the air flow path corresponds to reference numeral 409.

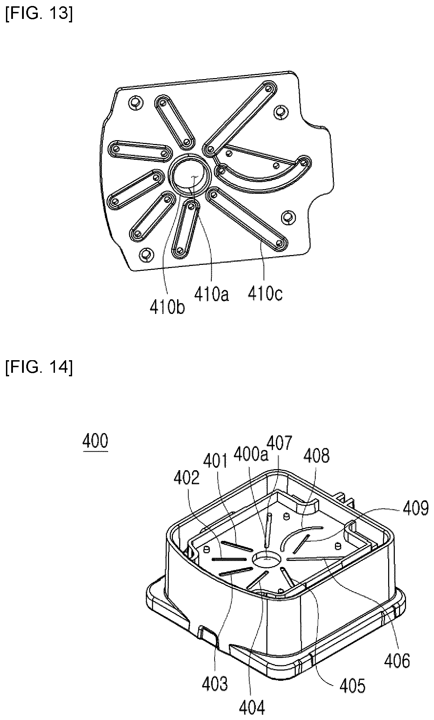

Referring to FIG. 14, some of the paths among the plurality of paths may have one end arranged on the same circumference, and the other end may also be arranged on the same circumference.

A piston driving unit insertion hole 400a is formed in the center of the base plate 400 so that the driving unit 800 that rotates the piston 700 may be coupled.

A flow cover 410 is placed in the mounting space on the upper part of the base plate 400. The flow cover 410 may be made of, for example, plastic, and may be formed integrally with the base plate 400 by ultrasonically welding while being mounted on the upper part of the base plate 400.

The flow cover 410 has a first through hole 410a aligned with the piston driving unit insertion hole 400a, and a plurality of first flow cover holes 411a, 412a, 413a, 414a, 415a, 416a, 417a, and 418a are formed in the flow cover 410 on a first circumference at a first distance from the first through hole 410a, and a plurality of second flow cover holes 411b, 412b, 413b, 414b, and 415b are formed in the flow cover 410 on a second circumference at a second distance from the first through hole 410a, and a plurality of third flow cover holes 416b, 417b, and 418b are formed in the flow cover 410 on a third circumference at a third distance from the first through hole 410a. In addition, a fourth flow cover holes 419a and 419b are formed in the flow cover 410. The fourth flow cover holes 419a and 419b communicate with one end and the other end of the air flow path 409, respectively. Here, the first flow cover holes are aligned with the inner ends of the flow paths formed in the base plate 400, the second flow cover holes and the third flow cover holes are aligned with the outer end of the flow path. The fourth flow cover holes communicate with one end and the other end of the air flow path. The second distance may be longer than the first distance and shorter than the third distance.

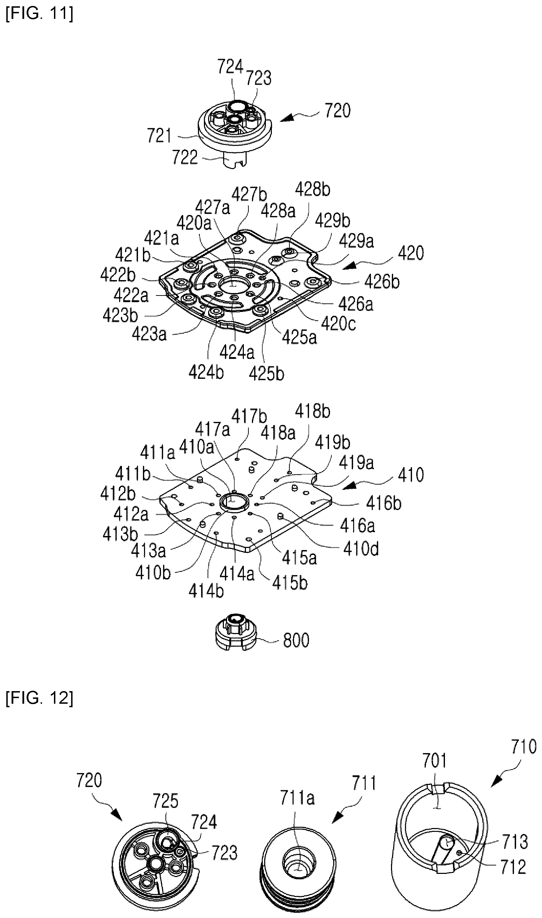

Referring to FIG. 11, a first coupling projection 410b that protrudes upwards and downwards on the outer periphery of the first through hole 410a may be further formed.

**In** addition, a molten projection 410c that is coupled along the edges of a plurality of flow paths of the base plate 400 may be formed protruding on the bottom surface of the flow cover 410 (see FIG. 12). When the flow cover 410 is installed on the upper surface of the base plate 400 and ultrasonic welding is performed, the molten projection 410c is melted and is integrated with the base plate 400. As such, integral coupling between the base plate 400 and the flow cover 410 can be achieved.

A pad 420 is placed on the flow cover 410. The pad 420 may be made of, for example, a silicone material, but any material having a predetermined elasticity may be applied without particular limitation.

A plurality of second coupling projections 410d protrude from the upper surface of the flow cover 410, and the plurality of second coupling projections 410d are coupled to coupling grooves 420c of the pad 420 so that firm coupling between the flow cover 410 and the pad 420 may be formed. In addition, the first coupling projection 410b of the flow cover 410 is also inserted into the second through hole 420a of the pad 420 so that firm coupling between the two components may be achieved.

The pad 420 has a second through hole 420a aligned with the first through hole 410a, and a plurality of first pad holes 421a, 422a, 423a, 424a, 425a, 426a, 427a, and 428a are formed in the pad 420 on the first circumference at a first distance from the second through hole 420a, and a plurality of second pad holes 421b, 422b, 423b, 424b, and 425b are formed in the pad 420 on the second circumference at a second distance from the second through hole 420a, and a plurality of third pad holes 426b, 427b, and 428b are formed in the pad 420 on the third circumference at a third distance from the second through hole 420a. In addition, fourth pad holes 429a and 429b are formed in the pad 420 and communicate with one end and the other end of the air flow path 409, respectively. Here, the first pad holes are aligned with the first flow cover holes, and the second pad holes are aligned with the second flow cover holes, and the third pad holes are aligned with the third flow cover holes, and the fourth pad holes are aligned with the fourth flow cover holes.

At portions of the upper surface of the pad 420 where a plurality of second pad holes 421b, 422b, 423b, 424b, and 425b, a plurality of third pad holes 426b, 427b, and 428b, and a fourth pad hole 429b are formed, protrusions may be further formed. By having such protrusions, even if the pad 420 is placed in close contact between the outer chamber 100 and the base plate 400, the problem of the diameter of the pad holes decreasing differently from the intended decrease may be solved.

The amplification module 600 is coupled to the outer chamber 100 and is configured to receive a specimen for which pre-treatment has been completed. The completion of the pre-treatment of the specimen means that the genome, such as Deoxyribo nucleic acid (DNA) or Ribo nucleic acid (RNA), contained in the specimen has been dissolved (lysis) into the reagent. When the genome extraction device 1000 according to the present invention is coupled to the diagnostic device (not shown), an amplification process (polymerase chain reaction (PCR), etc.) of the genome accommodated in the amplification module 600 may be performed.

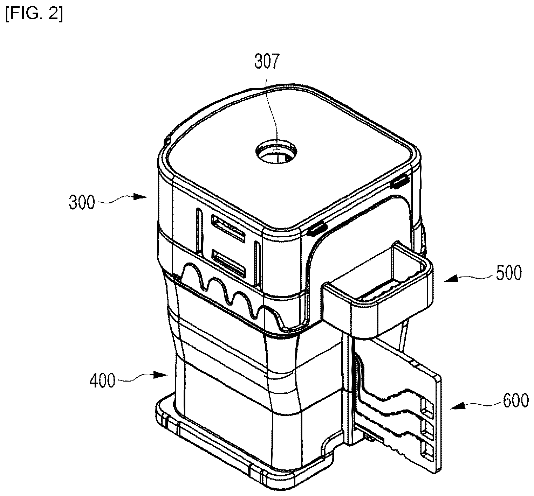

Referring to FIGS. 1 and 2, the amplification module 600 is coupled to the outer chamber 100 in a vertical direction. In other words, the amplification module 600 is coupled to the outer chamber 100 so that an upper part 631 of the receptacle 630 of the amplification module 600 is further away from the ground than a lower part 632 of the receptacle 630 of the amplification module 600.

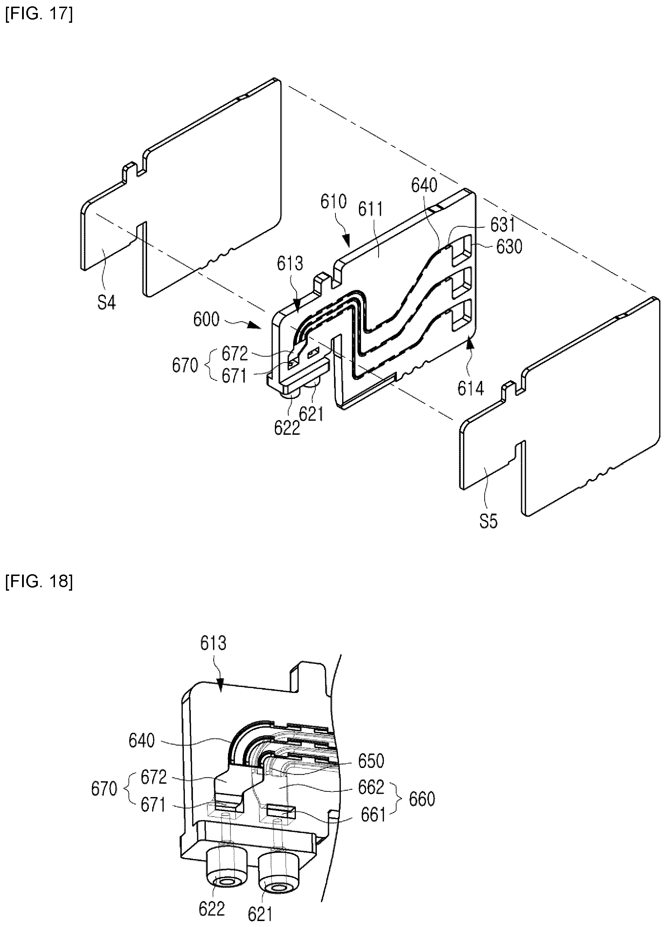

Referring to FIGS. 17 through 29, the amplification module 600 includes a body 610, an inlet 621, an outlet 622, a receptacle 630, a gas moving passage 640, and an extract moving passage 650.

The body 610 is a portion that constitutes the outer shape of the amplification module 600, and the inlet 621 and the outlet 622 are formed in a first end 613 of the body 610 which is coupled to the discharge holes 128 and 129 of the outer chamber 100.

The inlet 621 is coupled to the discharge hole 129 and serves as an inlet for the extract discharged from the discharge hole 128 to be injected into the receptacle 630, and the outlet 622 is coupled to the discharge hole 128 and serves as an outlet for the air inside to be discharged to the air flow path of the extraction device 1000 as the extract is injected into the amplification module 600.

That is, in a state in which the amplification module 600 is coupled to the extraction device 1000, the inlet 621 communicates with the liquid flow path 408, and the outlet 622 communicates with the air flow path 409.

The receptacle 630, which is a space for accommodating the extract introduced through the inlet 621, is formed at a position further from the extraction device 1000 than a second end 614 of the body 610, the inlet 621, and the outlet 622.

In an example, the receptacle 630 may be manufactured in a form that penetrates both one side and the opposite side of the body 610. However, in another example, the receptacle 630 may be manufactured in a form that penetrates only one side without passing through the opposite side. In both embodiments, the open sections are sealed by the sealing members S4 and/or S5. Thus, the extract and air are introduced into and discharged from the receptacle 630 only through the gas moving passage 640 and the extract moving passage 650.

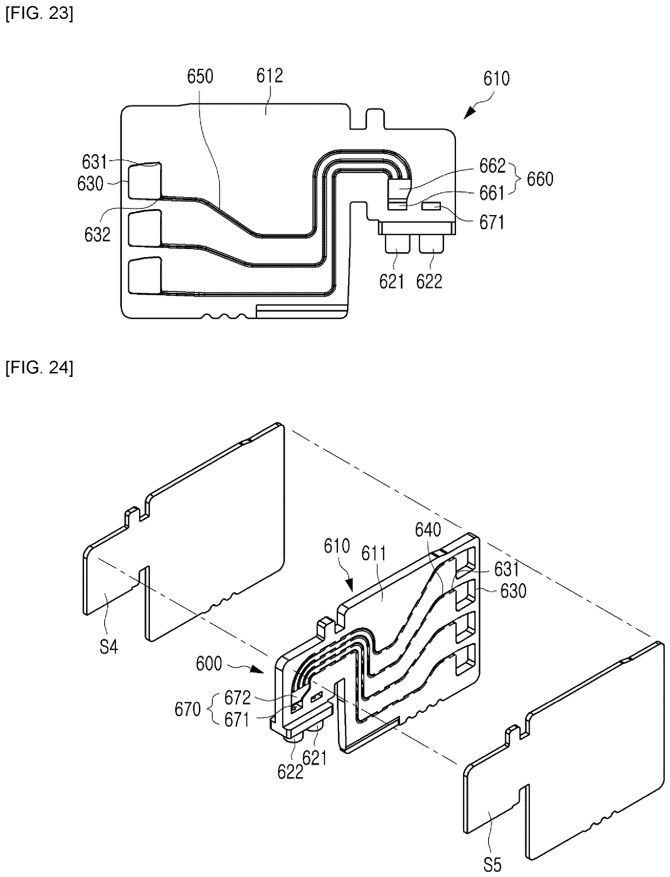

One or more receptacles 630 according to an embodiment of the present invention may be provided in one amplification module 600. FIGS. 17 through 23 illustrate an amplification module including three receptacles, and FIGS. 24 through 29 illustrate an amplification module including four receptacles.

The receptacle 630 may have an generally trapezoidal shape, and more specifically, preferably, the receptacle 630 may have a trapezoidal shape with rounded edges.

Here, the trapezoidal shape means a shape whose width becomes narrower as it gets farther away from the gas moving passage 640 and the extract moving passage 650. The receptacle 630 has the above shape so that the problem of bubbles being generated even if the extract is injected through the extract moving passage 650 is solved. When bubbles remain in the amplification module 600, especially in the receptacle 630, the problem of low analysis accuracy occurs in a fluorescence detection process after the amplification process, so that the problem may be solved through the shape of the receptacle 630. In the present invention, the amplification process may include an isothermal amplification process (Lamp) and a real-time nucleic acid amplification reaction (Real time Polymerase Chain Reaction), but the present invention is not particularly limited thereto as long as it is a process for a genome.

Primers and probes required for amplifying a genome are stored in the receptacle 630. The amplification module 600 according to the embodiment of the present invention includes one or more receptacles 630, and primers and probes targeting different substances may be provided in each receptacle 630. Thus, detection of various types of viruses/diseases may be performed simultaneously in the genome extracted from one sample. For example, primers and probes for amplifying a coronavirus may be provided in one receptacle 630, and primers and probes for amplifying an influenza virus may be provided in another receptacle 630, and different viruses/diseases with one amplification process in one amplification module 600 can be simultaneously detected.

According to an embodiment, the gas moving passage 640 may be formed on one surface 611 of the body 610 and configured to connect the outlet 622 to the upper part 631 of the receptacle 630. Contrary to this, the extract moving passage 650 may be formed on an opposite surface 612 opposite to the one surface 611 and configured to connect the inlet 621 to the lower part 632 of the receptacle 630.

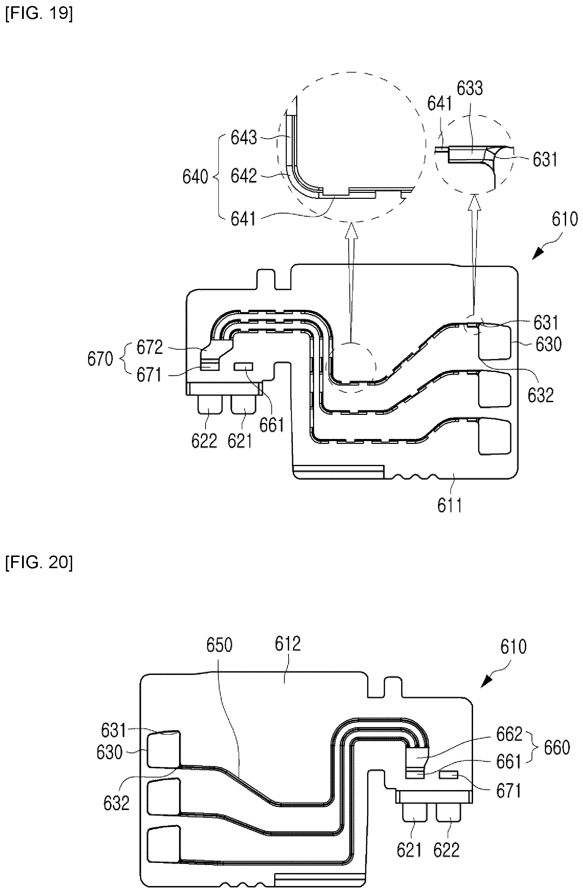

First, the extract moving passage 650 will be described with reference to FIGS. 20, 23, 26, and 29. As described above, the extract moving passage 650 serves as a passage through which the pre-treated extract moves in the genome extraction device 1000.

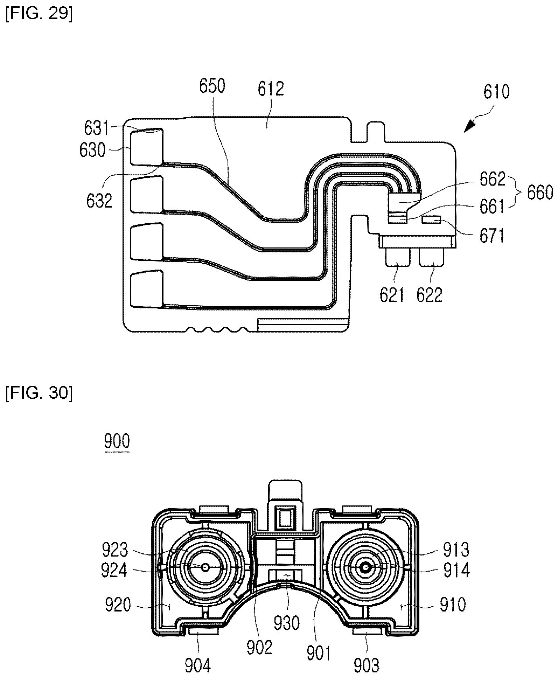

In an embodiment of the present invention, the number of extract moving passages 650 is the same as that of receptacles 630. In other words, in the case of an amplification module 600 including three receptacles 630 as in FIG. 20, three extract moving passages 650 are provided, and in the case of an amplification module 600 including four receptacles 630 as in FIG. 29, four extract moving passages 650 are provided.

Each extract moving passage 650 is configured so that the volumes of the passages is all the same. For example, each extract moving passage 650 may have the same length, width, and depth, and even if at least one of the depth, width, and depth is different, the capacity (volume) of each passage is configured to be the same. Thus, the extract spread along the extract moving passage 650 may reach the receptacles 630 at the same time, so that the extract may be filled in all the receptacles 630.

Meanwhile, in order to minimize the generation of bubbles during the process of the extract being distributed through the extract moving passage 650, the extract moving passage 650 is designed with curved connecting parts of the passage without angled parts to minimize bubble generation.

A first branch space 660 is formed between the extract moving passage 650 and the inlet 621. The first branch space 660 allows the inlet 621 and the extract moving passage 650 to communicate with each other.

Referring to FIG. 20, the first branch space 660 includes a first penetration portion 661 and a first recessed portion 662.

The first penetration portion 661 is configured to penetrate the body 610 while being connected to the inlet 621, and the first recessed portion 662 is formed between the first penetration portion 661 and the extract moving passage 650 without penetrating the body 610, but is configured to be recessed in one surface 611. As will be described later, a second recessed portion 672 is also formed on the opposite surface 612 of the body 610. Therefore, the first recessed portion 662 and the second recessed portion 672 are formed at positions where they partially overlap along the width direction. However, the space between the first recessed portion 662 and the sealing member, and the space between the second recessed portion 672 and the sealing member partially overlap so as to be independent spaces that do not communicate with each other. If the first recessed portion 662 and the second recessed portion 672 are configured to penetrate the body 610, the extract that is spread into the extract moving passage 650 through the inlet 621 may also be spread into the gas moving passage 640, and conversely, the air that is discharged from the receptacle 630 toward the outlet 622 may also be spread into the extract moving passage 650. Thus, in the present invention, the first recessed portion 662 and the second recessed portion 672 are configured in a recessed configuration, not a penetration configuration. Meanwhile, even if the first penetration portion and the second penetration portion, which do not overlap each other in the width direction, are formed in a penetration configuration, there is no interference problem.

Each extract moving passage 650 starts from the first recessed portion 662 and extends to the receptacle 630 while bending at least once. The width and depth of the first penetration portion 661 and the first recessed portion 662 are greater than the width and depth of the extract moving passage 650. Thus, assuming that the extract moving passage 650, the first penetration portion 661 and the first recessed portion 662 have the same length, the capacity (volume) of the first penetration portion 661 and the first recessed portion 662 is greater than that of the extract moving passage 650.

Therefore, the spreading speed of the extract through the inlet 621 is slower in the first branch space 660 than in the extract moving passage 650, causing a kind of stagnation phenomenon. After the first branch space 660 is completely filled with the extract, the extract may spread into the extract moving passage 650. This configuration has the advantage of allowing the extract to be distributed to each extract moving passage 650 simultaneously, compared to the case where the first branch space 660 is not provided. (In the case of an amplification module without the first branch space, the extract is first injected into the extract moving passage located at the lowest position due to gravity).

Meanwhile, preferably, the first branch space 660 may be formed at the first end 613 of the amplification module 600 in the width direction, and the receptacle 630 may be formed at the second end 614 opposite to the first end 613. In other words, preferably, the receptacle 630 and the first branch space 660 may be far apart in the width direction.

The extract is injected into and accommodated in the receptacle 630, and a heating device/cooling device is brought into close proximity or contact therewith to perform an amplification reaction. In order to prevent the amplification product in each receptacle 630 from being pushed out to the moving passages 640 and 650 by the heating temperature or flowing back to another receptacle 630 in advance during the amplification process, the first branch space 660 and the second branch space 670 described below are positioned at the first end 613 adjacent to the inlet 621 and the outlet 622.

The gas moving passage 640 serves as a passage through which gas (e.g., air) in the receptacle 630 moves. The flow path of the amplification module 600 is connected to the flow path of the genome extraction device 1000, forming an overall closed flow path. Since the receptacle 630 is filled with air before the extract is injected, if the extract is injected, an appropriate amount of air needs to be discharged to the outside. In the present invention, the air inside the amplification module 600 is discharged to the air flow path 409 through the outlet 622 via the gas moving passage 640, thereby preventing excessive pressure increase inside the receptacle 630 that occurs when the extract is injected, and solving the problem of bubbles that occur due to air remaining inside. Also, preferably, the gas moving passage 640 may be provided with curved connecting portions of the passage without angled portions, like the receptacle 630, to minimize the occurrence of bubbles.

Gas is lighter than a liquid such as an extract, and in the present invention, a plurality of gas moving passages 640 are connected to the upper end 631 of the receptacle 630. At the connection point between the receptacle 630 and the gas moving passage 640, a bubble receptacle 633 is located, which is wider and deeper than other parts of the other gas moving passages. Even if bubbles are generated inside as the extract is injected, the bubbles are accommodated in the wide and deep bubble receptacles 633 and 643, and thus, the bubbles do not affect the receptacle 630.

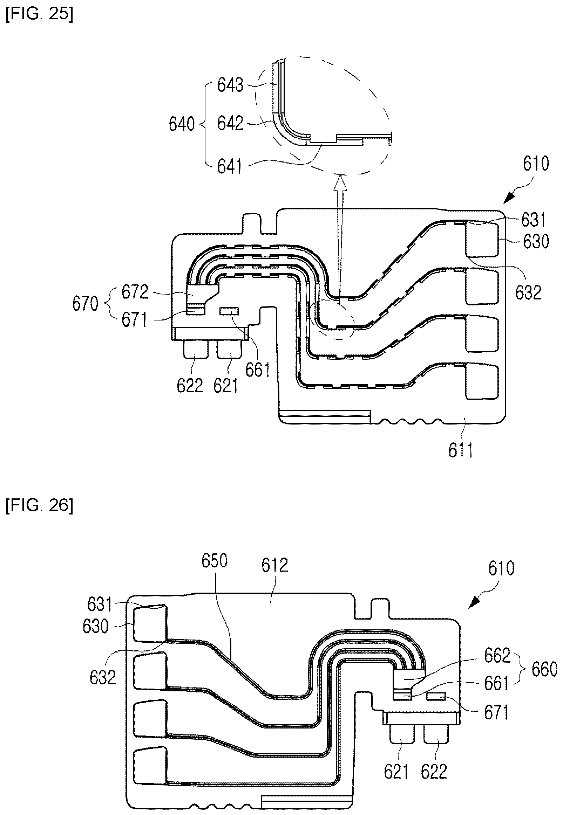

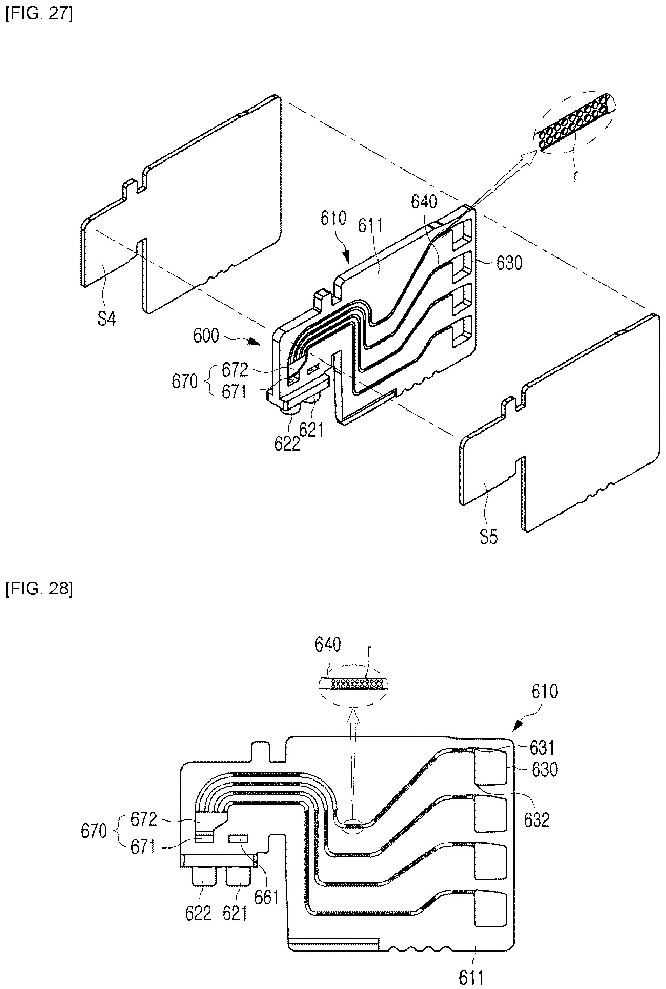

FIG. 25 illustrates a first embodiment of an amplification module having four receptacles 630, and FIG. 28 illustrates a second embodiment of an amplification module having four receptacles 630.

Both embodiments are characterized by minimizing the width and depth of the gas moving passage 640, so that liquid inflow into the gas moving passage 640 is minimized and only air can pass through.

The gas moving passage 640 of the amplification module 600 according to the first embodiment is configured through a combination of a first gas moving passage 641 having a first width and a first depth, a second gas moving passage 642 having a second width greater than the first width and a first depth, and a third gas moving passage 643 having a third width greater than the second width and a second depth deeper than the first depth. Specifically, the amplification module 600 according to the first embodiment is configured such that the volumes of each gas moving passage 640 are all the same.

Meanwhile, among the gas moving passages 640 of the amplification module 600 according to the first embodiment, the first gas moving passage 641 having the narrowest width and lowest depth may have a width of 0.135 mm to 0.165 mm, more specifically 0.14 mm to 0.16 mm, even more specifically 0.145 mm to 0.155 mm, and preferably 0.15 mm, and a depth of 0.045 mm to 0.055 mm, more specifically 0.0475 mm to 0.0525 mm, and preferably 0.05 mm.

In addition, the gas moving passage 640 of the amplification module 600 according to the second embodiment may have a constant width and depth along its longitudinal direction, and the width may be 0.45 mm to 0.55 mm, more specifically 0.475 mm to 0.525 mm, more specifically 0.49 mm to 0.51 mm, and preferably 0.5 mm, and the depth may be 0.045 mm to 0.055 mm, more specifically 0.0475 mm to 0.0575 mm, and more specifically 0.049 mm to 0.051 mm, and preferably 0.05 mm. In addition, the gas moving passage 640 of the amplification module 600 according to the second embodiment, is provided with a plurality of pillars r formed in a protruding form through a laser pattern processing method. Thus, the width and depth of the gas moving passage 640 are further minimized, so that liquid inflow is minimized and only air can pass through. As with the first embodiment, the amplification module 600 according to the second embodiment is also configured so that the volume of each gas moving passage 640 is the same.

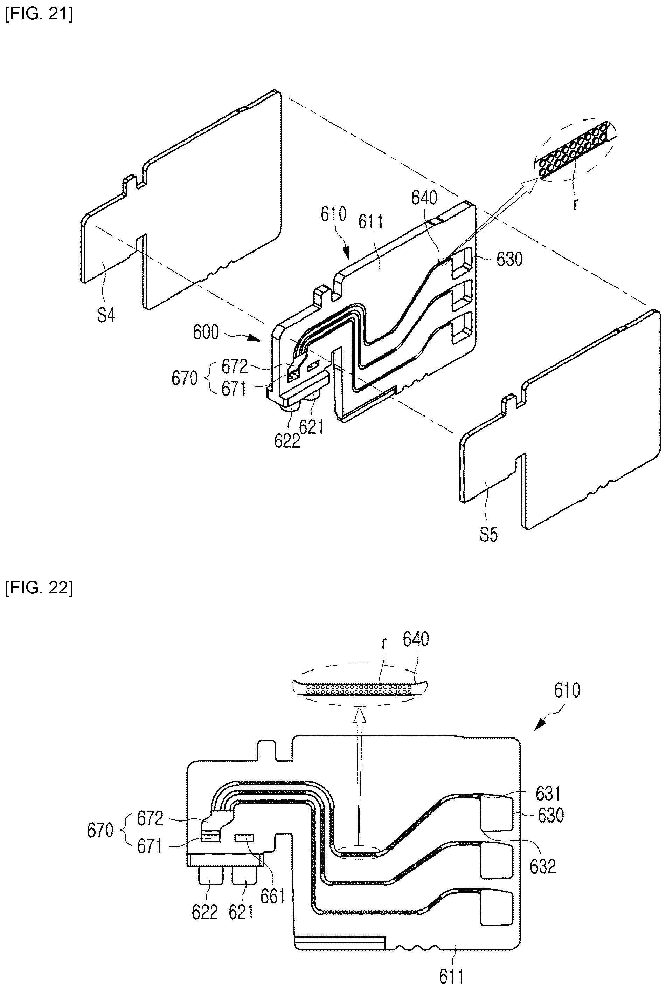

In the embodiment of the present invention, the number of gas moving passages 640 is the same as the number of receptacles 630. In other words, in the case of an amplification module 600 including three receptacles 630 as shown in FIGS. 19 and 22, three gas moving passages 640 are provided, and in the case of an amplification module 600 including four receptacles 630 as shown in FIGS. 25 and 28, four gas moving passages 640 are provided.

The piston 700 is inserted into the piston insertion portion 108 of the outer chamber 100 and is configured to inhale the reagent accommodated in the outer chamber 100 or discharge the reagent inhaled into the outer chamber 100 or the amplification module 600 according to the upward and downward movement.

Referring to FIGS. 3 and 12, the piston 700 includes an upper piston 710 and a lower piston 720.

The upper piston 710 has an open top, and a fluid receptacle 701 in which the inhaled fluids are accommodated, is formed inside the upper piston 710. A close-contact portion 711 is installed inside the upper piston 710. The outer surface of the close-contact portion 711 is in close contact with the inner surface of the upper piston 710, so that the fluid cannot enter or exit through the space between the outer surface of the close-contact portion 711 and the inner surface of the upper piston 710. A driving unit installation portion 711a to which a driving unit (not shown) of a diagnostic device is coupled is recessed in the center of the close-contact portion 711. The driving unit (not shown) of the diagnostic device is coupled to the driving unit installation portion 711a, and ascends and descends the close-contact portion 711 inside the upper piston 710 to inhale the fluid into the fluid receptacle 701 or discharge the fluid accommodated in the fluid receptacle 701 to the outside.

The bottom surface of the upper piston 710 is provided with a coupling structure that engages with the lower piston 720, and a first hole 712 connected to a liquid port of the lower piston 720 and a second hole 713 connected to a filter port of the lower piston 720 are formed in the upper piston 710. The second hole 713 may be formed to have a smaller diameter than a filter mounting space of the filter port so as to prevent detachment of a support structure and a filter.

The lower piston 720 is fixed by engaging with the coupling structure formed on the bottom surface of the upper piston 710.

The lower piston 720 may include a disc-shaped body 721, a shaft 722 formed to protrude outwards from the center of the body 721, and a liquid port 723 and a filter port 724 arranged at an equal distance from the center of the body 721.

The liquid port 723 is used when a specimen and a reagent are inhaled, mixed and discharged into the piston 700, and the filter port 724 may be used when a genome capture filter is washed or a genome is separated from the genome capture filter.

In addition, a groove recessed toward the center may be formed on the outer periphery of the body 721 of the lower piston 720. This groove serves to remove a vacuum that may occur when a liquid moves inside the extraction device.

The liquid port 723 and the filter port 724 are arranged at a certain angle apart from each other on the same circumference. For example, the two ports of the filter port 724 and the liquid port 723 may be arranged at an angle of 18 to 36 degrees, and more specifically, the two ports may be arranged at an angle of 22.5 degrees. When a step motor that performs one rotation by dividing into 16 times is used, the positions of the liquid port 723 and the filter port 724 may be changed by one driving.

The filter port 724 of the lower piston 720 may include a filter mounting space 725, and the filter and the support structure may be arranged in the filter mounting space 725. A glass fiber filter or a mold fixture having various particle sizes may be used as a filter for capturing the genome, and the support structure serves to fix the filter for capturing the genome.

The support structure may be formed of a porous plastic material having a constant particle size so as to prevent the filter from being detached when discharging the fluid and to maintain a constant pressure.

The driving unit 800 is connected to the driving unit (not shown) of the diagnostic device and serves as a medium to rotate the piston 700 at a certain angle.

The driving unit 800 may include a coupling groove formed in the center of one side of the driving unit 800 to engage with the shaft 722 and a driving groove formed in the other side of the driving unit 800 to engage with the driving unit (not shown) of the diagnostic device.

The driving unit 800 is coupled to the piston 700 and allows the liquid port 723 and the filter port 724 to be positioned at positions of the first discharge holes of the appropriate outer chamber 100 so that various chemical reactions required in the genome extraction step may be performed within one device.

The liquid port 723 and the filter port 724 are spaced apart at a certain angle, and the driving unit 800 rotates the ports to positions appropriate for each step during genome extraction.

The bead chamber 900 includes a first bead chamber 910, a second bead chamber 920, and a dehumidifying chamber 930, which are partitioned by a first bead chamber partition wall 901 and a second bead chamber partition wall 902. The first bead chamber 910 is inserted into the first space 106 of the outer chamber 100, and the second bead chamber 920 is inserted into the first space 107 of the outer chamber 100.

Similar to the inner chamber 200, the upper opening of the bead chamber 900 is also sealed by the third sealing member S3. The third sealing member S3 is perforated by the third protrusion members 316 and 317 formed on the bottom surface of the cover 300 when the cover 300 is coupled to the outer chamber 100. As the upper opening of the bead chamber 900 is opened by the third protrusion members 316 and 317, it becomes possible for a corresponding amount of air to be discharged through the perforated portion when fluid is introduced into the first bead chamber 910 and the second bead chamber 920.

The lower opening of the bead chamber 900 is provided in an open form without being separately sealed by a sealing member. Dry beads (more specifically, freeze-dried beads) are stored in the bead chamber 900, and dry beads have a characteristic of being vulnerable to moisture. In the genome extraction device according to the present invention, the lower opening of the bead chamber 900, the first space of the outer chamber 100, the flow cover 410, the pad 420, the flow path of the base plate 400, and the flow path of the amplification module 600 communicate with one another, but form a closed flow path that is not exposed to the outside air so that the inflow of moisture into the bead chamber 900 may be minimized.

A plurality of dry beads b1 required for genome extraction may be stored in the first bead chamber 910, and a plurality of dry beads b2 required for genome amplification may be stored in the second bead chamber 920.

A first bead holder 911 is installed in the upper opening of the first bead chamber 910 to prevent the dry beads b1 from being discharged while keeping them inside, and a first dehumidifying portion 912 is installed in the dehumidifying chamber 930 for dehumidifying the internal space of the first bead chamber 910. Here, the dry beads for the amplification of the genome material may be provided in the form of a capsule, for example, but the present invention is not particularly limited thereto.

A second bead holder 921 is installed in the upper opening of the second bead chamber 920 to prevent the dry beads b2 from being discharged while keeping them inside, and a second dehumidifying portion 922 is installed above the second bead holder 921 for dehumidifying the inside of the second bead chamber 920.

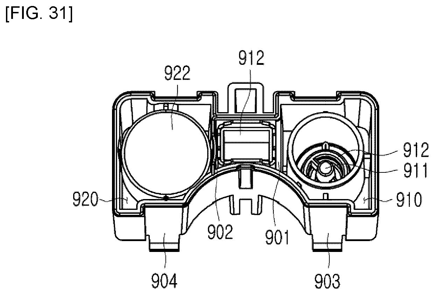

The third sealing member S3 seals the second bead chamber 920 so that the second bead chamber 920, the dehumidifying chamber 930 and the first bead chamber 910 do not communication with one another, but the first bead chamber 910 and the dehumidifying chamber 930 communicate with each other. This will be specifically described with reference to FIGS. 30 and 31.

The above-described effect is achieved through the height difference configuration between the first bead chamber partition wall 901 and the second bead chamber partition wall 902. Referring to FIGS. 30 and 31, the second bead chamber partition wall 902 that partitions the second bead chamber 920 and the dehumidifying chamber 930 has a higher height than the first bead chamber partition wall 901 that partitions the first bead chamber 910 and the dehumidifying chamber 930.

In other words, the upper part of the second bead chamber partition wall 902 extends to the same height as the upper part of the outer wall that defines the second bead chamber 920, and the upper part of the first bead chamber partition wall 901 extends to a height lower than the upper part of the outer wall that defines the first bead chamber 910.

Thus, even if the upper opening of the bead chamber 900 is sealed by the third sealing member S3, the first bead chamber 910 and the dehumidifying chamber 930 may communicate with each other through the space between the first bead chamber partition wall 901 and the third sealing member S3. Thus, the first bead chamber 910 is dehumidified by the second dehumidifying portion 922 installed inside the dehumidifying chamber 930.

A lower opening 914 of the first bead chamber 910 (i.e., the outlet of the first bead chamber) and a lower opening 924 of the second bead chamber 920 (i.e., the outlet of the second bead chamber) are formed at the lower ends of discharge passages 913 and 923 that become smaller as they go from the bead chamber 900 toward the base plate 400.

Dry beads may be accommodated inside the discharge passages 913 and 923, and bead holders may be installed on the upper portion of the discharge passages 913 and 923 to prevent external discharge of beads accommodated within them.

The discharge passages 913 and 923 may have a so-called tapered shape that becomes smaller as they go toward the base plate 400. The diameter of the lower openings 914 and 924 located at the end of the discharge passages 913 and 923 is provided to be smaller than the diameter of the dry beads so that the beads cannot be discharged to the outside through the lower openings 914 and 924. The fluid flows into the discharge passages 913 and 923 through the lower openings 914 and 942, the introduced fluid melts the dry beads, and may be discharged to the outside (fluid receptacle of the piston or amplification module) through the lower openings 914 and 924 only in the form of the fluid.

Here, the discharge passage 913 of the first bead chamber 910, in which the dry beads required for the genome amplification are stored, has a greater diameter than the discharge passage 923 of the second bead chamber 920 and may become smaller as it goes toward the base plate 400.

A configuration into which a last fluid is introduced before the pre-treated extract is injected into the amplification module 600, corresponds to the first bead chamber 910. Since accurate detection results can be obtained only when the fluid injected into the first bead chamber 910 does not remain in the first bead chamber 910 as much as possible and needs to be injected into the receptacle 630 of the amplification module 600, in the present invention, the discharge passage 913 of the first bead chamber 910 is formed to have a greater diameter than the discharge passage 923 of the second bead chamber 920 and becomes smaller so that the amount of fluid remaining in the first bead chamber 910 is minimized.

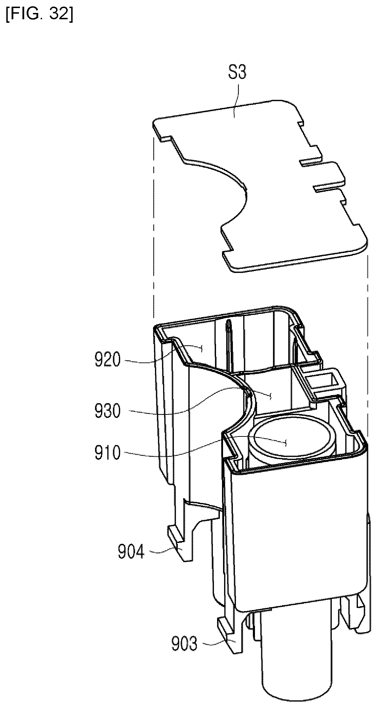

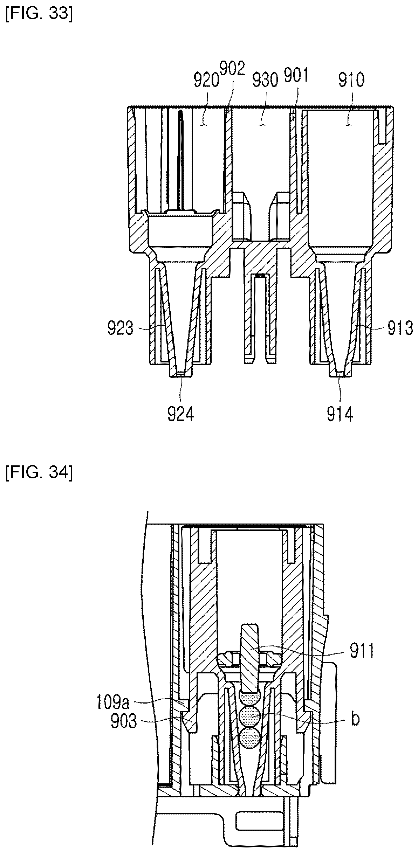

In addition, the bead chamber 900 according to the present invention has first catch protrusions 903 and 904 extending from the bottom surface of the outer partition walls of the first bead chamber 910 and the second bead chamber 920. As shown in FIGS. 32 and 34, the first catch protrusions 903 and 904 may be formed as a structure that extends toward the base plate 400 and protrudes outwards.

In the outer chamber 100 coupled to the bead chamber 900, a second catch protrusion 109a is formed on one side of the outer chamber partition wall that partitions a plurality of first spaces, and when force is applied to the bead chamber 900 toward the base plate 400, the first catch protrusions 903 and 904 pass the second catch protrusion 109a and are coupled to each other so that firm coupling between the two components may be achieved. When the first catch protrusions 903 and 904 are coupled to the second catch protrusion 109a, the relative position of the bead chamber 900 with respect to the outer chamber 100 is fixed.

Hereinafter, an extraction method according to an embodiment of the present invention will be specifically described.

First, (a) the inner chamber is coupled to the outer chamber through the upper openings of the plurality of first spaces of the outer chamber. Here, preferably, the fixing portion of the inner chamber may be coupled to the outer chamber while being coupled with the inner chamber coupling portion of the safety clip.

Next, (b) the cover is coupled to the outer chamber, and (c) the safety clip is removed from the outer chamber.

Next, (d) the cover is pressed, and the first sealing member that seals the upper opening of the inner chamber is torn by the first protrusion member formed on the bottom surface of the cover, and the second sealing member that seals the lower opening of the inner chamber is torn by the second protrusion member formed on the bottom surface of the plurality of first spaces of the outer chamber, so that the reagents accommodated in the inner chamber flow out into the plurality of first spaces, and (e) by the driving of the driving unit, the reagents that flow out into the plurality of first spaces are inhaled into and mixed in the fluid receptacle inside the upper piston, and then the mixed reagents are discharged to the amplification module.

The above operation (e) may include a plurality of sub-operations. Hereinafter, the operation (e) will be described in more detail.

First, (e1) a specimen to be analyzed is introduced into one of the plurality of first spaces of the outer chamber through the specimen introduction hole of the cover.

Next, (e2) a piston installed in the piston receptacle of the outer chamber rotates, so that the liquid port of the piston and the first discharge hole formed in the bottom surface of one of the first spaces into which the specimen to be analyzed is introduced, communicate with each other.

Next, (e3) a close-contact portion installed in the inner space of the piston rises so that the specimen to be analyzed accommodated in one of the first spaces is inhaled into the fluid receptacle inside the outer chamber.

Next, (e4) the piston rotates so that the liquid port of the piston and the first discharge hole formed in the bottom surface of another first space communicate with each other.

Next, (e5) the close-contact portion rises so that the first reagent accommodated in the other first space is inhaled into the fluid receptacle inside the outer chamber, so that the specimen to be analyzed and the first reagent are mixed in the fluid receptacle.

Next, (e6) the piston rotates so that the liquid port of the piston and the first discharge hole formed in the bottom surface of another first space communicate with each other.

Next, (e7) the close-contact portion rises so that the second reagent accommodated in the other first space is inhaled into the fluid receptacle inside the outer chamber so that the specimen to be analyzed, the first reagent, and the second reagent are mixed with one another.

Next, (e8) the piston rotates so that the filter port of the piston and the first discharge hole formed in the bottom surface of another first space communicate with each other.

Next, (e9) the close-contact portion descends so that the mixed solution accommodated in the fluid receptacle passes through the genome capture filter installed in the filter port and is discharged into another first space.

Next, (e10) the piston rotates so that the liquid port of the piston and the first discharge hole formed in the bottom surface of the first space in which the first reagent, the second reagent and other reagents are accommodated, communicate with each other.

Next, (e11) the close-contact portion rises, and other reagents are inhaled into and mixed in the fluid receptacle.

Next, (e12) the piston rotates so that the filter port of the piston and the first discharge hole formed in the bottom surface of the first space in which other reagents are accommodated, communicate with each other.

Next, (e13) the close-contact portion descends so that the mixed solution accommodated in the fluid receptacle passes through the genome capture filter and is discharged into the first space in which other reagents are accommodated.

Next, (e14) the piston rotates so that the liquid port of the piston and the first discharge hole formed in the bottom surface of the first space in which an eluent is accommodated, communicate with each other.

Next, (e15) the close-contact portion rises so that the eluent is inhaled into the fluid receptacle.

Next, (e16) the piston rotates so that the filter port of the piston and the second discharge hole formed in the bottom surface of the first space in which the beads required for genome amplification are accommodated, communicate with each other.

Next, (e17) the close-contact portion descends so that the eluent accommodated in the fluid receptacle passes through the genome capture filter and is discharged into the first space where beads required for genome amplification are accommodated, and the genome captured in the genome capture filter is separated from the genome capture filter and discharged together into the first space.

Next, (e18) the piston rotates so that the liquid port of the piston and the second discharge hole formed in the bottom surface of the first space where the genome is accommodated, communicate with each other.

Next, (e19) the close-contact portion rises so that the extract containing the genome is inhaled into the fluid receptacle.

Next, (e20) the piston rotates so that the liquid port of the piston and the amplification module communicate with each other.

Next, (e21) the close-contact portion descends so that the extract containing the genome accommodated in the fluid receptacle is discharged to the amplification module.

Next, (e22) the extract is introduced into the receptacle of the amplification module through the extract moving passage of the amplification module.

Next, (e23) the remaining air in the receptacle is discharged to the outside of the amplification module through the gas moving passage of the amplification module.

Next, (e24) the amplification device applies heat of a predetermined temperature or higher to the receptacle so that the genome is amplified.

Next, (e25) based on the fluorescence intensity of the amplification product of the genome, whether or not the specimen to be analyzed is infected with a disease is determined.

As described above, although the present specification has been described with reference to the embodiments illustrated in the drawings so that those skilled in the art can easily understand and reproduce the present invention, this is merely exemplary, and it will be understood by those skilled in the art that various modifications and equivalent other embodiments are possible from the embodiments of the present invention. Therefore, the protection scope of the present invention should be determined by the claims.

### [Explanation of Reference Numerals]

S1: first sealing member
S2: second sealing member
S3: third sealing member
S4, S5: sealing member
100: outer chamber
100a: outer surface upper portion
100b: outer surface lower portion
101, 102, 103, 104, 105, 106, 107: first space
108: piston insertion portion
109: mounting portion
109a: second catch projection
111, 112, 113, 114, 115: first protrusion member
111a, 112a, 113a, 114a, 115a: protrusion
111b, 112b, 113b, 114b, 115b: wing portion
119: second catch projection
121, 122, 123, 124, 125: first discharge hole
126, 127, 129: second discharge hole
128: air discharge hole
130: insertion space
131: stopper
200: inner chamber
201, 202, 203, 204, 205: second space
210: upper inner chamber
220: lower inner chamber
230: fixing portion
300: cover
301: cover body
302: lid
307: first insertion hole
308: second insertion hole
311, 312, 313, 314, 315: second protrusion member
316, 317: third protrusion member
320: separation member
400: base plate
400a: piston driving unit insertion hole
401, 402, 403, 404, 405, 406, 407, 408: liquid flow path
409: air flow path
410: flow cover
410a: first through hole
410b: first coupling projection
410c: molten projection
410d: second coupling projection
411a, 412a, 413a, 414a, 415a, 416a, 417a, 418a: first flow cover hole
411b, 412b, 413b, 414b, 415b: second flow cover hole
416b, 417b, 418b: third flow cover hole
419a, 419b: fourth flow cover hole
420: pad
420a: second through hole
421a, 422a, 423a, 424a, 425a, 426a, 427a, 428a: first pad hole
421b, 422b, 423b, 424b, 425b: second pad hole
426b, 427b, 428b: third pad hole
429a, 429b: fourth pad hole
420c: coupling groove
500: safety clip
510: outer chamber coupling portion
520: handle
530: upper extension portion
540: side extension portion
541: cover support member
542: inner chamber coupling portion
600: amplification module
610: body
611: one surface
612: opposite surface
613: first end
614: second end
621: inlet
622: outlet
630: receptacle
631: upper part
632: lower part
633: bubble receptacle
640: gas moving passage
641: first gas moving passage
642: second gas moving passage
643: third gas moving passage
650: first branch space
660: first recessed portion
661: first penetration portion
662: first recessed portion
670: second branch space
671: second penetration portion
672: second recessed portion
700: piston
701: fluid receptacle
710: upper piston
711: close-contact portion
711a: driving unit installation portion
712: first hole
713: second hole
720: lower piston
721: body
722: shaft
723: liquid port
724: filter port
800: driving unit
900: bead chamber
910: first bead chamber
911: first bed holder
912: dehumidifying portion
913: discharge passage
914: lower opening
920: second bead chamber
921: second bead holder
922: second dehumidifying portion
923: discharge passage
924: lower opening
930: dehumidifying chamber
1000: genome extraction device

## Claims

1. An amplification module comprising:
a body;
an inlet which is formed in the body and through which an extract is introduced;
a plurality of receptacles which are connected to the inlet and in which the introduced extract is accommodated;
a first branch space which communicates with the inlet;
a plurality of extract moving passages which are branched from the first branch space and connect the inlet and the plurality of receptacles to each other;
an outlet which is formed in the body and through which gas is discharged; and
a plurality of gas moving passages which connect the outlet and the plurality of receptacles to each other.

2. The amplification module of claim 1, wherein the first branch space comprises:
a first penetration portion which penetrates the body while being connected to the inlet; and
a first recessed portion which is formed between the first penetration portion and the plurality of extract moving passages and recessed in one surface of the body.

3. The amplification module of claim 2, wherein one or more of a width and a depth of the first branch space are wider or deeper than a width and a depth of the extract moving passages.

4. The amplification module of claim 1, wherein volumes of the plurality of extract moving passages are all the same.

5. The amplification module of claim 1, wherein the first branch space is formed in a first end of the amplification module in a width direction, and the plurality of receptacles are formed in a second end opposite to the first end of the amplification module in the width direction.

6. The amplification module of claim 2, further comprising a second branch space which is formed between the outlet and the plurality of gas moving passages,
wherein the second branch space comprises:
a second penetration portion which penetrates the body while being connected to the outlet; and
a second recessed portion which is formed between the second penetration portion and the plurality of gas moving passages and recessed in the other surface of the body.

7. The amplification module of claim 6, wherein the first recessed portion and the second recessed portion have a portion where they overlap partially in the width direction of the body and are recessed in the body.

8. The amplification module of claim 7, further comprising a sealing member which is attached to one surface of the body and an opposite surface to the one surface and is configured to seal the plurality of receptacles, the first branch space, the second branch space, the extract moving passages and the gas moving passages from an external space.

9. The amplification module of claim 8, wherein a space between the first recessed portion and the sealing member and a space between the second recessed portion and the sealing member do not communicate with each other.

10. The amplification module of claim 1, wherein the gas moving passage is connected to an upper part of the receptacle, and a space is formed in a connection point of the receptacle of the gas moving passage and is wider and deeper than other portions.

11. The amplification module of claim 1, wherein one or more of a width and a depth of the gas moving passage are narrower or shallower than a width and a depth of the extract moving passage.

12. The amplification module of claim 11, wherein the gas moving passage is formed through a combination of one or more comprising:
a first gas moving passage having a first width and a first depth;
a second gas moving passage having a second width greater than the first width and the first depth; and
a third gas moving passage having a third width greater than the second width and a second depth deeper than the first depth.

13. The amplification module of claim 11, wherein a plurality of pillars protrude from the gas moving passage.

14. The amplification module of claim 12 or 13, wherein gas moving passages connected to the plurality of receptacles all have the same capacity.

15. The amplification module of claim 1, wherein probes and primers for amplifying a first target material are stored in one of the plurality of receptacles, and probes and primers for amplifying a second target material different from the first target material are stored in another receptacle.
